# EUROPÄISCHE PATENTSCHRIFT

(11) **EP 1 983 918 B1**
(45) Veröffentlichungstag und Bekanntmachung des Hinweises auf die Patenterteilung: **14.10.2009**
(21) Anmeldenummer: 07703425.4
(22) Anmeldetag: 12.02.2007
(51) Int. Cl.: A61B 18/00

(54) **ELEKTROCHIRURGISCHES INSTRUMENT UND BAUREIHE FÜR ELEKTROCHIRURGISCHE INSTRUMENTE**
ELECTROSURGICAL INSTRUMENT AND TYPE SERIES FOR ELECTROSURGICAL INSTRUMENTS
INSTRUMENT ÉLECTROCHIRURGICAL ET GAMME D'INSTRUMENTS ÉLECTROCHIRURGICAUX

(30) Priorität: 14.02.2006 DE 102006006812
(43) Veröffentlichungstag der Anmeldung: 29.10.2008
(73) Patentinhaber: Erbe Elektromedizin GmbH, 72072 Tübingen (DE)
(72) Erfinder: BARTEL, Volker, 72411 Bodelshausen (DE)
(74) Vertreter: Bohnenberger, Johannes
(86) Internationale Anmeldenummer: PCT/EP2007/001196
(87) Internationale Veröffentlichungsnummer: WO 2007/093351

(56) Entgegenhaltungen:
- EP-A- 1 532 932
- EP-A- 1 568 330
- EP-A2- 0 904 738
- WO-A-97/12558
- DE-A1- 19 929 682
- JP-A- 8 154 491
- US-A1- 2004 006 340
- US-A1- 2004 249 374
- US-B1- 6 406 475

## Beschreibung

Die Erfindung betrifft ein elektrochirurgisches Instrument gemäß dem Oberbegriff des Patentanspruchs 1 bzw. eine Baureihe für elektrochirurgische Instrumente gemäß dem Oberbegriff des Patentanspruchs 13.

Bei der Hochfrequenz-Chirurgie (HF-Chirurgie) wird Wechselstrom mit hoher Frequenz durch den menschlichen Körper geleitet, um Gewebe gezielt zu schädigen bzw. zu schneiden. Ein wesentlicher Vorteil gegenüber herkömmlicher Schneidetechnik mit dem Skalpell ist, dass gleichzeitig mit dem Schnitt eine Blutungsstillung durch Verschluss der betroffenen Gefäße (Koagulation) erfolgen kann. Man unterscheidet zwischen einem Hochfrequenz-Generator, der den entsprechenden Wechselstrom erzeugt, und dem elektrochirurgischen Instrument, mittels dessen der Strom auf das Gewebe appliziert wird. Die häufig verwendeten elektrochirurgischen Instrumente sind elektrochirurgische Scheren und elektrochirurgische Klemmen. Diese Instrumente erfüllen eine gewisse Doppelfunktion, da sie zum einen ein herkömmliches mechanisches Klemmen und Schneiden und zusätzlich ein elektrisches Koagulieren und Schneiden durch die hochfrequenten Ströme gewährleisten.

Die bereits genannten elektrochirurgischen Instrumente gehören zur Klasse der bipolaren elektrochirurgischen Instrumente. Die Werkzeugköpfe der entsprechenden Instrumente weisen also zwei Pole auf, zwischen denen der Wechselstrom fließt. Am Beispiel der elektrochirurgischen Schere kann also ein erstes Scherblatt elektrisch getrennt von einem zweiten Scherblatt mit dem Wechselstrom versorgt werden. Der Stromkreis wird über das zu schneidende Gewebe zwischen den beiden Scherblättern geschlossen. Um eine gute Funktionalität dieser bipolaren elektrochirurgischen Instrumente zu gewährleisten, ist ein sicheres Isolieren der elektrischen Leiterbahnen in dem entsprechenden elektrochirurgischen Instrument notwendig. Hierbei erweisen sich die mechanischen Gelenke von herkömmlichen elektrochirurgischen Instrumenten als besonders problematisch, da es zu einer Überschneidung und Kontaktierung der entsprechenden Leiterbahnen kommt.

Diese beschriebenen elektrochirurgischen Instrumente sind hinreichend aus der US 6, 406,475 B1 und der US 5,324,289 bekannt. Um die jeweiligen Leiterbahnen voneinander zu trennen, wird dort vorgeschlagen, eine Isolation an den mechanischen Gelenken vorzusehen. Für eine sichere Isolierung der beiden Leiterbahnen werden hohe Anforderungen an diese Form der Isolierung gestellt, die in einer aufwendigen und auch teuren Fertigung resultieren.

Ein weiterer Nachteil der bekannten elektrochirurgischen Instrumente ist, dass die prozessgesteuerte Fertigung einer großen Produktpalette sehr aufwendig und teuer ist. So kann es beispielsweise sein, dass die Chirurgen gemäß der Anatomie ihrer Hände für das im Wesentlichen gleiche Instrument unterschiedliche Anforderungen an den zugehörigen Handgriff haben.

Ausgehend von diesem Stand der Technik ist es Aufgabe der vorliegenden Erfindung, ein elektrochirurgisches Instrument derart weiterzubilden, dass dieses sicher und funktional bedienbar sowie kostengünstig und effizient fertigbar ist.

Diese Aufgabe wird erfindungsgemäß durch ein elektrochirurgisches Instrument gemäß dem Anspruch 1 bzw. durch eine Baureihe gemäß dem Anspruch 13 gelöst.

Insbesondere wird die Aufgabe bei einem elektrochirurgischen Instrument, umfassend einen Handgriff mit einem ersten Griffteil und einem zweiten Griffteil und einem Werkzeugkopf mit einem ersten Werkzeugschenkel und einem zweiten Werkzeugschenkel, wobei der erste und zweite Werkzeugschenkel jeweils mit einem ersten Drehgelenk an einem zumindest teilweise isolierenden Gelenkelager befestigt sind, wobei das erste Griffteil mit dem ersten Werkzeugschenkel eine erste Leiterbahn und das zweite Griffteil mit dem zweiten Werkzeugschenkel eine zweite, von der ersten Leiterbahn elektrisch getrennte Leiterbahn bilden, dadurch gelöst, dass die Griffteile mit jeweils einem zweiten Drehgelenk an dem Gelenkelager kreuzungsfrei befestigt sind, die Werkzeugschenkel (11, 12) mit den zweiten Drehgelenken (35, 35') ebenfalls kreuzungsfrei befestigt sind, der erste Werkzeugschenkel durch eine erste Kupplung so an das erste Griffteil gekoppelt ist, dass eine Drehbewegung um das zweite Drehgelenk des ersten Griffteils in einer gegengerichteten Drehbewegung um das erste Drehgelenk des ersten Werkzeugschenkels resultiert und der zweite Werkzeugschenkel durch eine zweite Kupplung so an das zweite Griffteil gekoppelt ist, dass eine Drehbewegung um das zweite Drehgelenk des zweiten Griffteils in einer gegengerichteten Drehbewegung um das erste Drehgelenk des zweiten Werkzeugschenkel resultiert.

Der zentrale Gedanke der Erfindung besteht also darin, dass die einzelnen Leiterbahnen des elektrochirurgischen Instruments nebeneinander, quasi parallel, und somit automatisch getrennt angeordnet sind. Dieser Vorteil wird erzielt, indem weitere Drehgelenke vorgesehen sind, die eine mechanische Kreuzung der Griffteile und der Werkzeugschenkel vermeiden. Ein ähnliches Prinzip der Drehgelenke und Kupplungsanordnung ist von handelsüblichen Bolzenschneidern bekannt. Hierdurch kann zusätzlich eine vorteilhafte Kraftübertragung zwischen den Griffteilen und den zugehörigen Werkzeugschenkeln erzielt werden. Eine derartige Anordnung lässt sich einfach automatisiert fertigen und gewährleistet eine sichere Trennung der Leiterbahnen.

Es ist denkbar, die ersten Drehgelenke und die zweiten Drehgelenke so zu montieren, dass sie jeweils starr miteinander verbunden sind, jedoch die starren Verbindungen der ersten Drehgelenke gegenüber denen der zweiten Drehgelenke frei beweglich sind und die Werkzeugschenkel über eine nicht elastische Kupplung mit dem Griffteil verbunden sind. Vorteilhaft ist es aber, wenn die Drehgelenke alle starr miteinander verbunden sind. Eine bei einer Operation eventuell störende Längenveränderung des elektrochirurgischen Instruments, die durch die Drehgelenke und deren Bewegung bedingt ist, wird somit vermieden. Die starre Verbindung sollte so gewählt werden, dass die Drehbarkeit der Drehgelenke um deren jeweilige Achse nicht beeinflusst wird.

Eine vorteilhafte. Ausprägung des Gelenkelagers besteht darin, dass dieses zwei Isolatorplatten umfasst, zwischen denen die Drehgelenke angeordnet sind. Die Achsen der Drehgelenke stehen also im Wesentlichen senkrecht zu den Isolatorplatten. So können die Drehgelenke auf einfache Art und Weise ausgebildet werden, wobei eine sichere Isolation zwischen den einzelnen Drehgelenken, insbesondere zwischen dem ersten Drehgelenk des ersten Werkzeugschenkels gegen das erste Drehgelenk des zweiten Werkzeugschenkels sowie des zweiten Drehgelenks des ersten Griffteils gegen das zweite Drehgelenk des zweiten Griffteils, gewährleistet wird.

Vorzugsweise ist zwischen den Werkzeugschenkeln und zwischen den Griffteilen im Bereich der Drehgelenke ein Isolatorsteg zur Isolation der Leiterbahnen gegeneinander vorgesehen. Dieser Isolatorsteg erstreckt sich also im Wesentlichen parallel zu der ersten und der zweiten Leiterbahn zwischen den jeweiligen ersten und zweiten Drehgelenken. Die Isolation der Leiterbahnen gegeneinander wird somit erhöht. Des weiteren kann die erste und die zweite Kupplung so ausgebildet sein, dass eine mechanische Bewegung der ersten Kupplung auf die zweite Kupplung zu möglich ist. Um eine direkte Kontaktierung zu vermeiden, kann der Isolatorsteg als mechanische Trennung bzw. als ein Art Stopper fungieren.

Vorzugsweise ist der Isolatorsteg mit dem Gelenkelager, insbesondere mit mindestens einer der beiden Isolatorplatten verbunden. Das so entstehende T-Profil lässt sich einfach und einstückig fertigen. Zusammen mit der zweiten Isolatorplatte bildet sich ein H-Profil, wobei auf der einen Seite des Isolatorstegs die erste Leiterbahn und auf der anderen Seite die zweite Leiterbahn durch das isolierende H-Profil voneinander getrennt verlaufen.

Vorzugsweise umfasst das elektrochirurgische Instrument eine nicht leitende Synchronisationsvorrichtung, insbesondere zwischen den beiden ersten Drehgelenken und/oder zwischen den zweiten Drehgelenken, zur mechanischen Kupplung der ersten Drehgelenke. Die mechanische Kupplung bewirkt, dass eine Drehung des ersten Drehgelenks des ersten Werkzeugschenkels in einer Gegendrehung des ersten Drehgelenks des zweiten Werkzeugschenkels resultiert und umgekehrt. Die Synchronisationsvorrichtung sorgt also dafür, dass keiner der Werkzeugschenkel getrennt von dem anderen bewegt werden kann. Dies resultiert in einer besseren Bedienbarkeit des Instruments. Zur Ausbildung dieser Synchronisationsvorrichtung kann mitunter der Isolatorsteg verwendet werden, wobei dieser vorzugsweise Vorrichtungen zur Aufnahme von an den ersten Drehgelenken befestigten Zahnrädern aufweist.

Es können zwar mehrere Leiterbahne in dem ersten und dem zweiten Griffteil vorgesehen werden, vorteilhaft ist es aber, wenn jedes Griffteil je einen elektrischen Anschluss für je eine elektrische Leiterbahn umfasst.

Vorzugsweise umfassen die Werkzeugschenkel je eine Nut, in die jeweils ein Vorsprung des Griffteils zur Bildung der ersten bzw. der zweiten Kupplung eingreift. Die Kupplung wird also als ein offenes Gelenk ausgebildet, wobei bei der Bewegung der Werkzeugschenkel der Vorsprung des ersten Griffteils in die Nut des ersten Werkzeugschenkels und der Vorsprung des zweiten Griffteils in die Nut des zweiten Werkzeugschenkels unterschiedlich tief eingreift. Diese Kupplung kann jeweils einzahnig ausgebildet sein.

Alternativ können die Drehgelenke Zahnräder umfassen, deren Drehpunkt mit den Drehachsen des jeweiligen Drehgelenks zusammenfallen. Als Kupplung dienen dann die ineinander greifenden Zahnräder. Hierbei greift das Zahnrad des ersten Drehgelenks des ersten Werkzeugschenkels in das Zahnrad des zweiten Drehgelenks des Griffteils und das Zahnrad des ersten Drehgelenks des zweiten Werkzeugschenkels in das Zahnrad des zweiten Drehgelenks des zweiten Griffteils ein.

Vorzugsweise sind die Kupplungen aus elektrisch leitendem Material gefertigt und bilden einen Teil der ersten bzw. der zweiten Leiterbahn aus. Somit dient die mechanischen Kupplung gleichzeitig als elektrische Kupplung und der Stromkreis wird einfach und sicher ausgebildet.

Es ist vorteilhaft, wenn der Werkzeugkopf und der Handgriff aus Metall gefertigt und teilweise mit einer Isolationsschicht überzogen sind. Die einzelnen Teile des Werkzeugkopfs und des Handgriffs sind somit in ihrem Inneren leitend und können zur Bildung der ersten bzw. zweiten Leiterbahn verwendet werden. Die Isolationsschicht schützt vor ungewollten elektrischen Kurzschlüssen.

Erfindungsgemäß wird die zuvor gestellte Aufgabe auch durch eine Baureihe für elektrochirurgische Instrumente gelöst. Hierbei umfasst die Baureihe mindestens zwei Werkzeugköpfe und/oder mindestens zwei Handgriffe, die wechselseitig über die Drehgelenke und die Kupplung miteinander verbindbar sind.

Der zentrale Gedanke besteht also, eine Art Baukastensystem bereitzustellen, bei dem verschiedene unterschiedliche Werkzeugköpfe mit unterschiedlichen Handgriffen kombiniert werden können. Die Verbindung dieser beiden Elemente erfolgt über die Drehgelenke und die Kupplungen.

Vorzugsweise sind die Griffteile, insbesondere eine Griffteillänge, der unterschiedlichen Handgriffe unterschiedlich ausgebildet. So kann beispielsweise für den gleichen Werkzeugkopf je nach Beschaffenheit des Griffteils unterschiedlich viel Druck aufgebracht werden. Auch ist es denkbar, mögliche Fingerlöcher an den Griffteilen zu variieren, um ein individuelles Instrument für den Chirurgen zu fertigen.

Vorzugsweise umfassen die Werkzeugköpfe mindestens einen Werkzeugkopf zum Schneiden und mindestens einen Werkzeugkopf zum Abklemmen. Bei der maschinellen Fertigung können so für unterschiedliche elektrochirurgische Instrumente, wie Schere und Klemme, eine Vielzahl von gleichen Bauelementen verwendet werden. Der Fertigungsprozess wird günstiger, die Instrumente können besser individualisiert werden.

Nachfolgend wird die Erfindung anhand von Ausführungsbeispielen beschrieben, die mittels Abbildungen näher erläutert werden. Hierbei zeigen:
- Fig. 1a eine erfindungsgemäße elektrochirurgische Klemme;
- Fig. 1b den Werkzeugkopf der elektrochirurgischen Klemme aus Fig. 1a;
- Fig. 1c eine Detailansicht auf das Gelenkelager der elektrochirurgischen Klemme aus Fig. 1a;
- Fig. 2 die elektrochirurgische Klemme aus Fig. 1a im geschlossenen Zustand;
- Fig. 3a die elektrochirurgische Klemme aus Fig. 1a mit geschlossenem Kuppellager; und
- Fig. 3b eine Detailansicht auf das geschlossene Kuppellager der Fig. 3a.

In den nachfolgenden Beschreibungen werden für gleiche und gleich wirkende Teile die selben Bezugsziffern verwendet.

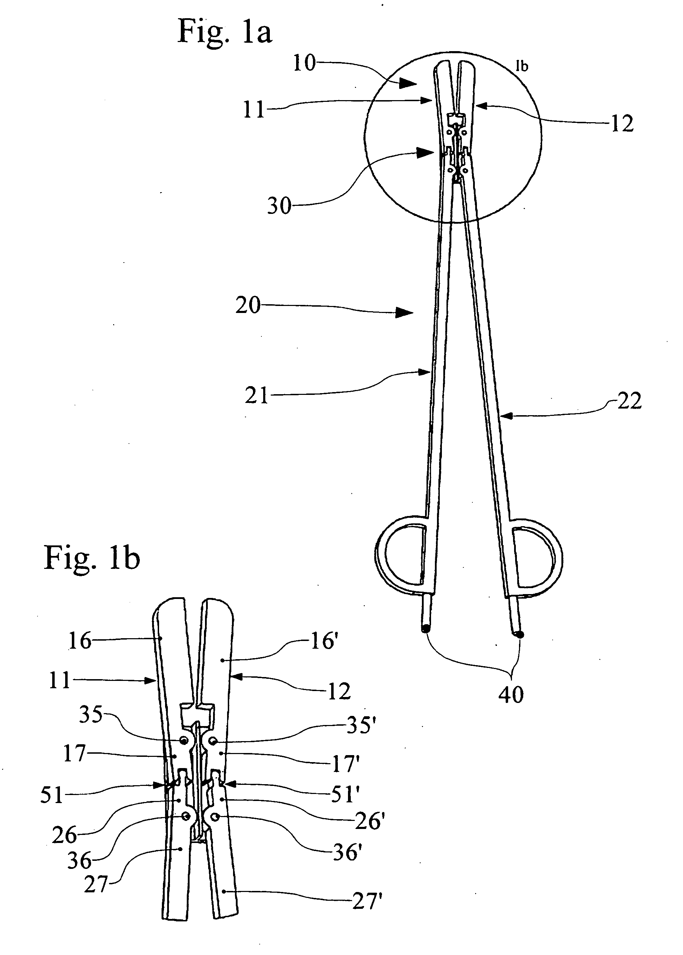

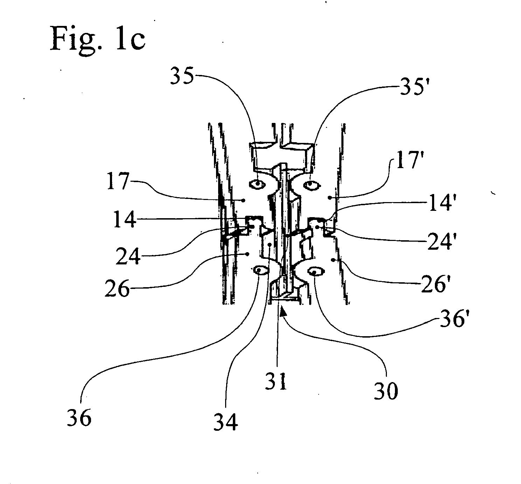

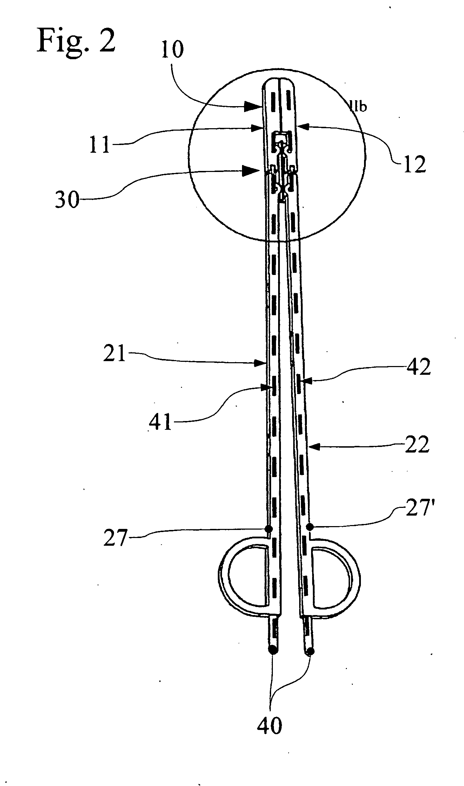

Fig. 1a zeigt eine erfindungsgemäße elektrochirurgische Klemme. Das Instrument umfasst einen Handgriff 20 mit einem ersten Griffteil 21 und einem zweiten Griffteil 22 zum Halten des elektrochirurgischen Instruments in der Hand und einen Werkzeugkopf 10 zum Greifen und Abklemmen von Gewebe. Der Handgriff 20 und der Werkzeugkopf 10 sind über ein isolierendes Gelenkelager 30 miteinander starr verbunden. Um den Aufbau des Gelenklagers zu verdeutlichen, ist es in den Figuren 1a, 1b, 1c, 2 geöffnet dargestellt. Der Werkzeugkopf 10 umfasst einen ersten Werkzeugschenkel 11 und einen zweiten Werkzeugschenkel 12, die an den dem Gelenkelager entfernten Seiten je eine Greiffläche zum Aufnehmen des Gewebes umfassen. Bei der erfindungsgemäßen Klemme handelt es sich um ein bipolares elektrochirurgisches Instrument. D.h. der Werkzeugkopf, genauer gesagt, die Greifflächen bilden zwei voneinander elektrisch getrennte Pole aus. Diese Pole werden mittels einer ersten Leiterbahn 41 (vgl. Fig. 2) und einer zweiten Leiterbahn 42 an den elektrischen Stromkreis eines Hochfrequenz-Generators angeschlossen. Wie in Fig. 2 schematisch gekennzeichnet, verlaufen diese Leiterbahnen 41, 42 voneinander getrennt parallel entlang der Längsrichtung des elektrochirurgischen Instruments bzw. der Klemme. Um dies zu gewährleisten, sind die Werkzeugschenkel 11, 12 und die Griffteile 21, 22 aus elektrisch leitenden Material ausgebildet. Für den elektrischen Anschluss an den Hochfrequenzgenerator weisen die Griffteile 21, 22 je einen elektrischen Anschluss 40 auf.

Zur definierten Bewegung des elektrochirurgischen Instruments hat die Klemme vier Drehgelenke 35, 35', 36, 36', die am Gelenkelager 30 angebracht sind (vgl. Fig. 1b, 1c). Der erste Werkzeugschenkel 11 bildet um dessen erstes Drehgelenk 35 eine wippenartige Vorrichtung. Das erste Drehgelenk 35 stellt den Drehpunkt dar, um den ein Vorderwerkzeugabschnitt 16 und ein Hinterwerkzeugabschnitt 17 des ersten Werkzeugschenkels 11 drehbar sind. Dieser Hinterwerkzeugabschnitt 17 steht mit einem Vordergriffabschnitt 26 des ersten Griffteils 21 in Eingriff. Ähnlich wie der erste Werkzeugschenkel 11 bildet auch das erste Griffteil 21 ein wippenähnliche Vorrichtung, bei der der Vordergriffabschnitt 26 und ein Hintergriffabschnitt 27 um das zweite Drehgelenk 36 des ersten Griffteils 21 drehbar gelagert sind.

Der zweite Werkzeugschenkel 12 und das zweite Griffteil 22 sind im Wesentlichen spiegelsymmetrisch zu dem ersten Werkzeugschenkel 11 und dem ersten Griffteil 21 ausgebildet und am Gelenkelager 30 angeordnet. So umfasst der zweite Werkzeugschenkel 12 einen Vorderwerkzeugabschnitt 16' und einen Hinterwerkzeugabschnitt 17', die um das erste Drehgelenk 35 drehbar gelagert sind. Das zweite Griffteil 22 weist auf der einen Seite des zweiten Drehgelenks 36' einen Vordergriffabschnitt 26' und auf der anderen Seite einen Hintergriffabschnitt 27' auf. Der Hintergriffabschnitt 17' des zweiten Werkzeugschenkels 12 steht mit dem Vordergriffabschnitt 26' des zweiten Griffteils 22 in Eingriff.

Bewegt man die Hintergriffabschnitte 27, 27' in dieser symmetrischen Anordnung aufeinander zu, so kommt es zu einer Rotationsbewegung um die zweiten Drehgelenke 36, 36'. Die Rotationsbewegung der beiden zweiten Drehgelenke 36, 36' sind einander entgegengerichtet orientiert. Mittels einer ersten Kupplung 51 und einer zweiten Kupplung 52 wird diese Rotationsbewegung invertiert und auf die beiden ersten Drehgelenke 35, 35' übertragen. Die Vorderwerkzeugabschnitte 16, 16' werden aufeinander zubewegt. Der Werkzeugkopf 10 wird geschlossen.

Erfindungsgemäß lässt sich die Kraftübertragung des ersten Griffteils 21 auf den ersten Werkzeugschenkel 11 und des zweiten Griffteils 22 auf den zweiten Werkzeugschenkel 12 durch die Variation der Länge der Vordergriffabschnitte 26, 26' für jede beliebige Anwendung anpassen. Natürlich muss die Länge der Hinterwerkzeugabschnitte 17, 17' entsprechend gewählt werden.

Erfindungsgemäß umfasst die erste Kupplung 51 und die zweite Kupplung 52 je eine Nut 14 bzw. 14' sowie einen Vorsprung 24 bzw. 24', die ineinander eingreifen (vgl. Fig. 1 c). Um bei den bereits beschriebenen Rotationsbewegungen der Drehgelenke 35, 35', 36, 36' eine Kontaktierung der ersten Kupplung 51 mit der zweiten Kupplung 52 zu verhindern, umfasst das Gelenkelager 30 einen Isolierungssteg 31. Dieser Isolierungssteg 31 steht senkrecht zu einem Lagerboden 34 und bildet mit diesem ein T-Profil. Der Isolierungssteg 31 erstreckt sich so mittig zwischen den Kupplungen 51, 52, dass eine Kontaktierung dieser verhindert wird und die Leiterbahnen 41, 42 stets gegeneinander isoliert sind.

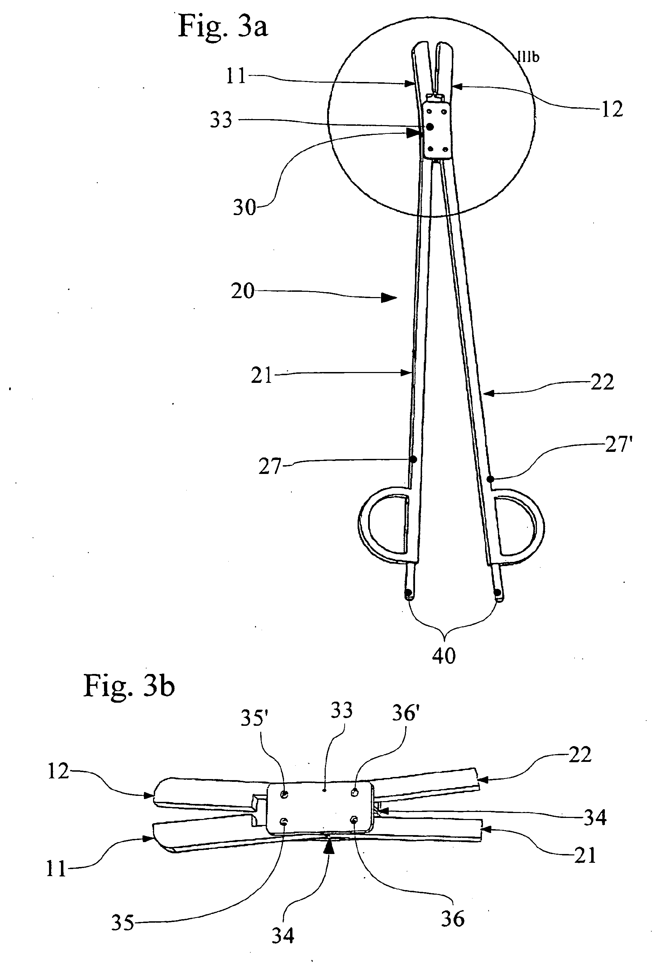

In Fig. 3a wird wiederum die erfindungsgemäße medizinische Klemme der Figuren 1a und 2 gezeigt. Das Gelenkelager 30 ist hier aber verschlossen. Den Verschluss bildet ein Lagerdeckel 33.

Wie aus den Figuren 3a, 3b ersichtlich, setzt sich das Gelenkelager 30 aus dem Lagerdeckel 33, dem Lagerboden 34 und dem Isolatorsteg 31 zusammen. Der Lagerdeckel 33 ist parallel zu dem Lagerboden 34 angeordnet. Somit bilden in einem verschlossenen Zustand des Gelenkelagers 30 diese Elemente ein H-Profil.

Der Aufbau der einzelnen Drehgelenke 35, 35', 36, 36' ist jeweils gleich. Durch eine Öffnung in dem Lagerboden 34 ist ein Bolzen durch das jeweilige Griffteil 21, 22 bzw. den jeweiligen Werkzeugschenkel 11, 12 zu einer Öffnung im Lagerdeckel 33 geführt. Die Werkzeugschenkel 11, 12 und Griffteile 21, 22 sind also um den jeweiligen Bolzen drehbar. Da das gesamte Gelenkelager 30 aus elektrisch isolierendem Material, beispielsweise aus Kunststoff, gefertigt ist, sind die beiden Leiterbahnen 41, 42 elektrisch gegeneinander abgeschirmt.

In einem weiteren Ausführungsbeispiel kann das Gelenkelager 30 nur teilweise aus elektrisch isolierendem Material gefertigt sein, und einen Teil der ersten und zweiten Leiterbahn 41, 42 ausbilden. In diesem Ausführungsbeispiel weist der Lagerdeckel 33 zwei im Wesentlichen parallele elektrisch leitende Bereiche auf. Der erste elektrisch leitende Bereich verläuft zwischen dem ersten Drehgelenk 35 des ersten Werkzeugschenkels zum zweiten Drehgelenk 36 des ersten Griffteils 21, der zweite Bereich vom ersten Drehgelenk 35' des zweiten Werkzeugschenkels 12 zum zweiten Drehgelenk 36' des zweiten Griffteils 22. In der ersten Leiterbahn 41 fließt also der elektrische Strom zuerst durch das erste Griffteil 21, dann abschnittsweise durch das Gelenkelager 30 zu dem ersten Werkzeugschenkel 11. Analog fließt in der zweiten Leiterbahn 42 der Strom durch das zweite Griffteil 22, einen Abschnitt des Gelenkelagers 30 und den zweiten Werkzeugschenkel 12. Der elektrische Kontakt zwischen den Werkzeugschenkeln 11, 12 und dem Gelenkelager 30, insbesondere dem jeweiligen elektrisch leitenden Bereich des Lagerdeckels 33, wird durch direkte Kontäktierung sowie den Bolzen der Drehgelenke 35, 35', 36, 36' hergestellt. Auf analoge Weise wird auch der elektrische Kontakt zwischen den Griffteilen 21, 22 und dem Gelenkelager 30 bzw. dem elektrisch leitenden Bereich des Lagerdeckels 33 bereitgestellt.

In einem weiteren Ausführungsbeispiel sind die ersten Drehgelenke synchronisiert. Hierfür ist der Isolierungssteg 31 nicht mit dem Rest des Gelenkelagers 30 verbunden. Stattdessen weist der Isolierungssteg 31 entlang seiner Längsrichtung auf beiden Seiten Zähne auf, in die an den ersten Drehgelenken 35, 35' angebrachte Zahnräder eingreifen. Bei der Bewegung der Griffteile 21, 22 bzw. der angekoppelten Werkzeugschenkel 11, 12 drehen sich die Zahnräder der ersten Drehgelenke 35, 35' um die Drehachse der Drehgelenke 35, 35' und verschieben den Isolierungssteg 31 innerhalb des Gelenkelagers 30 entlang der Längsrichtung des Isolierungsstegs 31. Jede Drehbewegung eines der beiden ersten Drehgelenke 35, 35' wird somit automatisch auf das andere der ersten Drehgelenke 35, 35' übertragen.

In einem weiteren Ausführungsbeispiel ist das elektrochirurgische Instrument im Wesentlichen baugleich der vorhergehenden Ausführungsbeispiels ausgebildet. Aufgrund einer anderen Ausbildung des Werkzeugkopfes 10, insbesondere der Vorderwerkzeugabschnitte 16, 16', eignet sich dieses elektrochirurgische Instrument besonders zum Durchtrennen bzw. Schneiden von Gewebe. Anders als die zuvor beschriebene elektrochirurgische Klemme weist der Werkzeugkopf keine Greifflächen auf, sondern Scherblätter, die ein mechanisches Schneiden ermöglichen. Bei einer Operation wirken so die mechanischen Effekte und die elektrischen Effekte Hand in Hand und ermöglichen das gleichzeitige Durchtrennen und Veröden bzw. Koagulieren von Gewebe.

### Bezugszeichenliste

- 10: Werkzeugkopf
- 11: erster Werkzeugschenkel
- 12: zweiter Werkzeugschenkel
- 14,14': Nut
- 16, 16': Vorderwerkzeugabschnitt
- 17, 17': Hinterwerkzeugabschnitt
- 20: Handgriff
- 21: erstes Griffteil
- 22: zweites Griffteil
- 24, 24': Vorsprung
- 26, 26': Vordergriffabschnitt
- 27, 27': Hintergriffabschnitt
- 30: Gelenkelager
- 31: Isolierungssteg
- 33: Lagerdeckel
- 34: Lagerboden
- 35, 35': erstes Drehgelenk
- 36, 36': zweites Drehgelenk
- 40: elektrische Anschlüsse
- 41: erste Leiterbahn
- 42: zweite Leiterbahn
- 51: erste Kupplung
- 52: zweite Kupplung

## Patentansprüche

1. Elektrochirurgisches Instrument, umfassend
einen Handgriff (20) mit einem ersten Griffteil (21) und einem zweiten Griffteil (22) und
einen Werkzeugkopf (10) mit einem ersten Werkzeugschenkel (11) und einem zweiten Werkzeugschenkel (12),
wobei der erste und zweite Werkzeugschenkel (11, 12) jeweils mit einem ersten Drehgelenk (35, 35') an einem zumindest teilweise isolierenden Gelenkelager (30) befestigt sind,
wobei das erste Griffteil (21) mit dem ersten Werkzeugschenkel (11) eine erste Leiterbahn (41) und
das zweite Griffteil (22) mit dem zweiten Werkzeugschenkel (12) eine zweite, von der ersten Leiterbahn (41) elektrisch getrennte Leiterbahn (42) bilden,
**dadurch gekennzeichnet, dass**
die Griffteile (21, 22) mit jeweils einem zweiten Drehgelenk (36, 36') an dem Gelenkelager (30) kreuzungsfrei befestigt sind;
die Werkzeugschenkel (11, 12) mit den ersten Drehgelenken (35, 35') ebenfalls kreuzungsfrei befestigt sind;
der erste Werkzeugschenkel (11) durch eine erste Kupplung (51) so an das erste Griffteil (21) gekoppelt ist, dass eine Drehbewegung um das zweite Drehgelenk (36) des ersten Griffteils (21) in einer gegengerichteten Drehbewegung um das erste Drehgelenk (35) des ersten Werkzeugschenkels (11) resultiert; und
der zweite Werkzeugschenkel (12) durch eine zweite Kupplung (52) so an das zweite Griffteil (22) gekoppelt ist, dass eine Drehbewegung um das zweite Drehgelenk (36') des zweiten Griffteils (22) in einer gegengerichteten Drehbewegung um das erste Drehgelenk (35') des zweiten Werkzeugschenkels (12) resultiert.

2. Elektrochirurgisches Instrument nach Anspruch 1,
**dadurch gekennzeichnet, dass**
die Drehgelenke (35, 35', 36, 36') starr miteinander verbunden sind.

3. Elektrochirurgisches Instrument nach einem der vorhergehenden Ansprüche,
**dadurch gekennzeichnet, dass**
das Gelenkelager (30) zwei Isolatorplatten (33, 34) umfasst, zwischen denen die
Drehgelenke (35, 35', 36, 36') angeordnet sind.

4. Elektrochirurgisches Instrument nach einem der vorhergehenden Ansprüche,
**dadurch gekennzeichnet, dass**
zwischen den Werkzeugschenkeln (21, 22) und zwischen den Griffteilen (11, 12) im Bereich der Drehgelenke (35, 35', 36, 36') ein Isolatorsteg (31) zur Isolation der Leiterbahnen (41, 42) gegeneinander vorgesehen ist.

5. Elektrochirurgisches Instrument nach einem der vorhergehenden Ansprüche, insbesondere nach Anspruch 4,
**dadurch gekennzeichnet, dass**
der Isolatorsteg (31) mit dem Gelenkelager (30) verbunden ist.

6. Elektrochirurgisches Instrument nach einem der vorhergehenden Ansprüche,
**gekennzeichnet durch**
eine nicht leitende Synchronisationsvorrichtung, insbesondere zwischen den beiden ersten Drehgelenken (35, 35') und/oder zweiten Drehgelenken (36, 36'), zur mechanischen Kopplung der ersten Drehgelenke (35, 35').

7. Elektrochirurgisches Instrument nach einem der vorhergehenden Ansprüche,
**dadurch gekennzeichnet, dass**
das erste und das zweite Griffteil (21, 22) je einen elektrischen Anschluss (40) umfassen.

8. Elektrochirurgisches Instrument nach einem der vorhergehenden Ansprüche,
**dadurch gekennzeichnet, dass**
die Werkzeugschenkel (11, 12) je eine Nut (14,14') umfassen, in die jeweils ein Vorsprung (24, 24') der Griffteile (21, 22) zur Bildung der ersten bzw. der zweiten Kupplung (51, 52) eingreift.

9. Elektrochirurgisches Instrument nach einem der vorhergehenden Ansprüche, insbesondere nach Anspruch 8,
**dadurch gekennzeichnet, dass**
die Nut (14, 14') und der Vorsprung (24, 24') entsprechend einer Zahnradverzahnung ausgebildet sind.

10. Elektrochirurgisches Instrument nach einem der vorhergehenden Ansprüche,
**dadurch gekennzeichnet, dass**
die Kupplungen (51, 52) aus elektrisch leitendem Material gefertigt sind und einen Teil der ersten Leiterbahn bzw. der zweiten Leiterbahn (41, 42) ausbilden.

11. Elektrochirurgisches Instrument nach einem der vorhergehenden Ansprüche,
**dadurch gekennzeichnet, dass**
die ersten Drehgelenke (25, 25', 26, 26') jeweils eine Aufnahmevorrichtung zur lösbaren Befestigung des Werkzeugkopfes (10) an dem Gelenkelager (30) umfassen.

12. Elektrochirurgisches Instrument nach einem der vorhergehenden Ansprüche,
**dadurch gekennzeichnet, dass**
der Werkzeugkopf (10) und der Handgriff (20) aus Metal gefertigt und teilweise mit einer Isolationsschicht überzogen sind.

13. Baureihe für elektrochirurgische Instrumente nach einem der vorhergehenden Ansprüche,
**gekennzeichnet durch**
mindestens zwei Werkzeugköpfe (10) und/oder mindestens zwei Handgriffe (20), die wechselseitig über die Drehgelenke (35, 35', 36, 36') und die Kuppelungen (51, 52) miteinander verbindbar sind.

14. Baureihe nach Anspruch 13,
**dadurch gekennzeichnet, dass**
die Griffteile (21, 22), insbesondere eine Griffteillänge, der Handgriffe (20) unterschiedlich ausgebildet sind.

15. Baureihe nach einem der Ansprüche 13 oder 14,
**dadurch gekennzeichnet, dass**
die Werkzeugköpfe (10) mindestens einen Werkzeugkopf (10) zum Schneiden und mindestens einen Werkzeugkopf (10) zum Abklemmen umfassen.

## Claims

1. Electrosurgical instrument, comprising
a handle (20) with a first handle member (21) and a second handle member (22) and a tool head (10) with a first tool limb (11) and a second tool limb (12),
wherein the first and second tool limb (11, 12) are each fastened with a first pivot joint (35, 35') to an at least partially insulated pivot joint (30),
wherein the first handle member (21) together with the first tool limb (11) forms a first conducting path (41)
and the second handle member (22) forms, with the second tool limb (12), a second conducting path (42), which is electrically separated from the first conducting path (41),
**characterised in that**
the handle members (21, 22) are each attached with a second pivot joint (36, 36') to the joint bearing (30) without crossing over;
that the tool limbs (11, 12) are also fastened with the second pivot joints (35, 35') without crossing over,
the first tool limb (11) is coupled via a first coupling (51) to the first handle member (11) such that a pivot movement about the second pivot joint (36) of the first handle member (21) results in a contrary rotary movement about the first pivot joint (35) of the first tool limb (11); and
the second tool limb (12) is coupled via a second coupling (52) to the second handle member (22) such that a pivot movement about the second pivot joint (36') of the second handle member (22) results in a contrary rotary movement about the first pivot joint (35') of the second tool limb (12).

2. Electrosurgical instrument according to claim 1,
**characterised in that**
the pivot joints (35, 35', 36, 36') are rigidly connected to one another.

3. Electrosurgical instrument according to one of the preceding claims,
**characterised in that**
the joint bearing (30) comprises two insulator plates (33, 34) between which the pivot joints (35, 35', 36, 36') are arranged.

4. Electrosurgical instrument according to one of the preceding claims,
**characterised in that**
an insulator web (31) is provided between the tool limbs (21, 22) and between the handle members (11, 12) in the region of the pivot joints (35, 35', 36, 36'), in order to insulate the conducting paths (41, 42) relative to one another.

5. Electrosurgical instrument according to one of the preceding claims, in particular claim 4,
**characterised in that**
the insulator web (31) is connected to the joint bearing (30).

6. Electrosurgical instrument according to one of the preceding claims,
**characterised by**
a non-conducting synchronisation device, in particular between the two first pivot joints (35, 35') and/or between the second pivot joints (36, 36'), for mechanical coupling of the first pivot joints (35, 35').

7. Electrosurgical instrument according to one of the preceding claims,
**characterised in that**
the first and second handle member (21, 22) each comprise an electrical terminal (40).

8. Electrosurgical instrument according to one of the preceding claims,
**characterised in that**
the tool limbs (11, 12) each comprise a groove (14, 14'), in which a projection (24, 24') of the handle members (21, 22) engages to form the first or second coupling (51, 52).

9. Electrosurgical instrument according to one of the preceding claims, in particular claim 8,
**characterised in that**
the groove (14, 14') and the projection (24, 24') are configured according to gear toothing.

10. Electrosurgical instrument according to one of the preceding claims,
**characterised in that**
the couplings (51, 52) are made from electrically conductive material and comprise a part of the first or second conducting path (41, 42).

11. Electrosurgical instrument according to one of the preceding claims,
**characterised in that**
the first pivot joints (25, 25', 26, 26') each comprise a receptacle device for detachable fastening of the tool head (10) to the joint bearing (30).

12. Electrosurgical instrument according to one of the preceding claims,
**characterised in that**
the tool head (10) and the handle (20) are made from metal and are partially covered with an insulating layer.

13. Type series for electrosurgical instruments according to one of the preceding claims,
**characterised by**
at least two tool heads (10) and/or at least two handles (20), which are mutually connectable via the pivot joints (35, 35', 36, 36') and the couplings (51, 52).

14. Type series according to claim 13,
**characterised in that**
the handle members (21, 22), and particularly a handle member length of the handles (20) are configured to be different.

15. Type series according to claim 13 or 14,
**characterised in that**
the tool heads (10) preferably comprise at least one tool head (10) for cutting and at least one tool head (10) for clamping.

## Revendications

1. Instrument électrochirurgical, comprenant :
une poignée (20) présentant une première partie de poignée (21) et une seconde partie de poignée (22) et
une tête d'outil (10) présentant une première branche d'outil (11) et une seconde branche d'outil (12),
dans lequel les première et seconde branches d'outil (11, 12) sont fixées, chacune par le biais d'une première articulation à rotation (35, 35'), à un support d'articulation au moins partiellement isolant (30),
la première partie de poignée (21) formant avec la première articulation d'outil (11) une première piste conductrice (41) et
la seconde partie de poignée (22) formant avec la seconde articulation d'outil (12) une seconde piste conductrice (42) qui est séparée au plan électrique de la première piste conductrice (41),
**caractérisé en ce que**
les parties de poignée (21, 22) sont fixées chacune par le biais d'une seconde articulation à rotation (36, 36') au support d'articulation (30) sans croisement ;
les branches d'outil (11, 12) sont fixées aux premières articulations à rotation (35, 35') également sans croisement ;
la première branche d'outil (11) est couplée par le biais d'un premier couplage (51) à la première partie de poignée (21) de sorte qu'un mouvement de rotation autour de la seconde articulation à rotation (36) de la première partie de poignée (21) puisse se faire selon un mouvement de rotation en sens opposé à celui se produisant autour de la première articulation à rotation (35) de la première branche d'outil (11) ; et la seconde branche d'outil (12) est couplée par le biais d'un second couplage (52) à la seconde partie de poignée (22) de sorte qu'un mouvement de rotation autour de la seconde articulation à rotation (36') de la seconde partie de poignée (22) puisse se faire selon un mouvement de rotation en sens opposé de celui se produisant autour de la première articulation à rotation (35') de la seconde branche d'outil (12).

2. Instrument électrochirurgical selon la revendication 1,
**caractérisé en ce que**
les articulations à rotation (35, 35', 36, 36') sont mutuellement reliées de manière rigide.

3. Instrument électrochirurgical selon l'une quelconque des revendications précédentes,
**caractérisé en ce que**
le support d'articulation (30) comprend deux plaques isolantes (33, 34), entre lesquelles les articulations à rotation (35, 35', 36, 36') sont aménagées.

4. Instrument électrochirurgical selon l'une quelconque des revendications précédentes,
**caractérisé en ce que**
l'on a prévu entre les branches d'outil (21, 22) et entre les parties de poignée (11, 12), dans la zone des articulations à rotation (35, 35', 36, 36'), une barrette isolante (31) dans le but d'isoler les pistes conductrices (41, 42) l'une de l'autre.

5. Instrument électrochirurgical selon l'une quelconque des revendications précédentes, en particulier selon la revendication 4,
**caractérisé en ce que**
la barrette isolante (31) est fixée au support d'articulation (30).

6. Instrument électrochirurgical selon l'une quelconque des revendications précédentes,
**caractérisé par**
un dispositif de synchronisation non conducteur, en particulier, entre les deux premières articulations à rotation (35, 35') et/ou les deux secondes articulations à rotation (36, 36'), pour le couplage mécanique des premières articulations à rotation (35, 35').

7. Instrument électrochirurgical selon l'une quelconque des revendications précédentes,
**caractérisé en ce que**
la première et la seconde parties de poignée (21, 22) comprennent, respectivement, une connexion électrique (40).

8. Instrument électrochirurgical selon l'une quelconque des revendications précédentes,
**caractérisé en ce que**
les branches d'outil (11, 12) comprennent, respectivement, une rainure (14,14'), dans laquelle s'enclenche respectivement une saillie (24, 24') des parties de poignée (21, 22) pour la formation du premier ou du second couplage (51, 52).

9. Instrument électrochirurgical selon l'une quelconque des revendications précédentes, en particulier selon la revendication 8,
**caractérisé en ce que**
la rainure (14, 14') et la saillie (24, 24') sont formées à la manière d'une denture de roue dentée.

10. Instrument électrochirurgical selon l'une quelconque des revendications précédentes,
**caractérisé en ce que**
les couplages (51, 52) sont fabriqués en matériau électroconducteur et forment une partie de la première piste conductrice ou de la seconde piste conductrice (41, 42).

11. Instrument électrochirurgical selon l'une quelconque des revendications précédentes,
**caractérisé en ce que**
les premières articulations à rotation (25, 25', 26, 26') comprennent respectivement un dispositif de réception pour fixation amovible de la tête d'outil (10) au support d'articulation (30).

12. Instrument électrochirurgical selon l'une quelconque des revendications précédentes,
**caractérisé en ce que**
la tête d'outil (10) et la poignée (20) sont fabriquées en métal et sont partiellement revêtues d'une couche isolante.

13. Gamme de fabrication pour instruments électrochirurgicaux selon l'une quelconque des revendications précédentes,
**caractérisée par**
au moins deux têtes d'outil (10) et/ou au moins deux poignées (20), qui peuvent être fixées mutuellement de manière interchangeable sur les articulations à rotation (35, 35', 36, 36') et sur les couplages (51, 52).

14. Gamme de fabrication selon la revendication 13,
**caractérisé en ce que**
les parties de poignée (21, 22), en particulier une longueur de partie de poignée, des poignées (20) sont élaborées de manière différente.

15. Gamme de fabrication selon l'une quelconque des revendications 13 ou 14,
**caractérisée en ce que**
les têtes d'outil (10) comprennent au moins une tête d'outil (10) pour la coupe et au moins une tête d'outil (10) pour le retrait par pinçage.
